(19) Europäisches Patentamt
European Patent Office
Office européen des brevets

(11) Publication number: 0 143 840
B1

(12) # EUROPEAN PATENT SPECIFICATION

(45) Date of publication of patent specification: 29.08.90

(51) Int. Cl.$^5$: C 07 H 17/00, C 08 B 37/00

(21) Application number: 84902242.1

(22) Date of filing: 02.05.84

(86) International application number: PCT/US84/00670

(87) International publication number: WO 84/04526 22.11.84 Gazette 84/27

(54) ## MONOSACCHARIDE COMPOUNDS HAVING IMMUNOSTIMULATING ACTIVITY.

(30) Priority: 06.05.83 US 492378

(43) Date of publication of application: 12.06.85 Bulletin 85/24

(45) Publication of the grant of the patent: 29.08.90 Bulletin 90/35

(84) Designated Contracting States: CH DE FR GB LI

(56) References cited:

Nishijima et al., Two interacting Mutations Causing Temperature-Sensitive Phosphastidylglycerol Synthesis in Escherichia coli Membranes, Journal of Bacteriology 145(1), pp. 113-131 (1981)

Nishijima et al., Membrane Lipid Biogenesis in Escherichia coli: Identification of Genetic Loci for Phosphatidylglycerophosphate Synthetase and Construction of Mutants Lacking Phosphatidylglycerol, Journal of Biological Chemistry, 254(16), pp. 7837-44 (1979)

The file contains technical information submitted after the application was filed and not included in this specification

(73) Proprietor: WISCONSIN ALUMNI RESEARCH FOUNDATION
614 North Walnut Street
Madison, WI 53705 (US)

(72) Inventor: RAETZ, Christian, R., H.
6705 Maywood Avenue
Middleton, WI 53562 (US)
Inventor: ANDERSON, Laurens
5639 Lake Mendota Drive
Madison, WI 53705 (US)

(74) Representative: Newell, William Joseph et al
Wynne-Jones, Lainé & James 22 Rodney Road
Cheltenham GL50 1JJ (GB)

(56) References cited:
Nishijima et al., Characterization of Two Membrane Associated Glycolipids from an Escherichia coli Mutant Deficient in Phosphatidylglycerol, Journal of Biological Chemistry 256 (20),pp. 10690-96 (1981)

Qureshi et al., Purification and Structural Determination of Nontoxic Lipid A Obtained from the Lipopolysaccharide of Salmonella Typhimurium, Journal of Biological Chemistry 257 (19), pp. 11808-15 (1982)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 143 840 B1

(56) References cited:

Rick et al., Lipid A Mutants of Salmonella Typhimurium, Journal of Biological Chemistry 252 (14), pp. 4004-12 (1977)

Lehmann, Isolation, Purification and Properties of an Intermediate in 3-deoxy-D-manno-octulosonic acid - lipid A Biosynthesis, Chemical Abstracts 87: 18670z (1977)

Chiller et al., Relation of the Structure of Bacterial Lipopolysaccharides to its Function in Mitogenesis and Adjuvanticity, Chemical Abstracts 79: 102658u (1973)

Shiba, Synthetic Approach to Elucidation for Chemical Structure and Biological Activity of Lipid A, Chemical Abstracts 97: 119894n (1982)
Kasai et al., Basal Structure, especially the Molecular Weight, of the Lipid Moiety of Endotoxins, Chemical Abstracts 75:33334p (1970)

CARBOHYDRATE RESEARCH, no. 95, 1981, pp. C5-C8; Elsevier Scientific Publishung Co., Amsterdam, NL; MAKOTO KISO et al.:"A new synthetic route to 2-(acylamino)-2-deoxy-alpha-D-glucopyranosyl phosphates, and their endotoxic activity related to the Salmonella-type lipid A."

CHEMICAL ABSTRACTS, vol. 95, no. 11, September 14, 1981, p. 697, ref. no. 98176w; Columbus, Ohio, US; M. KISO et al.:"Synthesis of 2-(acylamino)-2-deoxy-alpha-D-gluco-pyranosyl phosphates: monosaccharide analogs of lipid A."

CHEMICAL ABSTRACTS, vol. 97, no. 3, July 19, 1982, p. 541, ref. no. 21842u; Columbus, Ohio, US; T. YASUDA et al.:"Biological activity of chemically synthesized analogs of lipid A. Demonstration of adjuvant effect in hapten-sensitized liposomal system"

CHEMICAL ABSTRACTS, vol. 99, no. 15, October 10, 1983, p. 483, ref. no. 120441k; Columbus, Ohio, US; S. KOTANI et al.:"Immunobiological activities of synthetic lipid A analogs and related compounds as compared with those of bacterial lipopolysaccharide, Re-glycolipid, lipid A, and muramyl dipeptide."

TETRAHEDRON LETTERS, vol.22, no. 24, pp. 2281-2284, 1981; Pergamon Press Ltd., GB; MASARU INAGE et al.: "Chemical synthesis of phosphorylated fundamental structure of lipid A."

**Description**

The present invention relates to a pharmaceutical composition comprising an immunostimulating amount of a compound, the use of a compound in the preparation of an immunostimulating agent, a method of stimulating the activity of immune cells of an animal *in vitro*, and certain glucosamine derivatives.

Lipid A is a component of the bacterial lipopolysaccharide which is a complex amphipathic molecule which covers the outer surface membrane of *Escherichbia coli* and other gram-negative bacteria. Lipid A is a unique hydrophobic anchor substance which holds the lipopolysaccharide molecule in place.

The exact structure of lipid A is still unknown; however, the components include a β,1→6-linked glucosamine disaccharide, 2—3 phosphate groups, 6—7 fatty acids, an aminoarabinose, and ethanolamine. The lipid is structurally heterogeneous because the polar aminoarabinose and phosphoryl-ethanolamine residues occur in only a fraction of the molecules. Microheterogeneity based on the ester-linked fatty acids is also indicated by the results of a study of the purified monophosphoryl lipid. The fatty acid composition of lipid A differs from that of the conventional phospholipids by the presence of β-hydroxymyristic acid and the near absence of unsaturated fatty acids.

There is considerable interest in lipid A, as well as its precursors and its metabolites because of its biological activity. Lipid A is believed to be responsible for the endotoxic, immunostimulating, tumor cell killing, and interferon production stimulating activities of the lipopolysaccharides. A comprehensive review of the chemistry and biology of lipid A can be found in the C. Galanos et al. article which appears in the "International Review of Biochemistry, Biochemistry of Lipids II", Volume 14, *University Park Press* (1977).

Substantial work has been done to determine the molecular structure of lipid A and to identify the portions of the lipid A molecule which are responsible for the biological activity.

In an article in the *Journal of Biological Chemistry,* Vol. 254, No. 16, pp. 7837—7844 (1979) Masahiro Nishijima and Christian R. H. Raetz noted the presence of two unidentified lipids (X and Y) which accumulated in certain *Escherichia coli* mutants defective in phosphatidylglycerol synthesis, and appeared "to be metabolites of lipopolysaccharide synthesis," pp. 7837. In a later article in *Journal of Bacteriology,* Vol. 145, No. 1, pp. 113—121 (1981), Nishijima, Raetz and Christine E. Bulawa characterized the lipids X and Y as "precursors of lipid A biosynthesis", p. 118. Still later, Nishijima and Raetz, in an article in *The Journal of Biological Chemistry,* Vol. 256, No. 20, pp. 10690—10696 (1981) purified milligram quantities of lipids X and Y from their *Escherichia coli* mutants defective in phosphatidylglycerol synthesis, and they speculated that the lipids X and Y were disaccharides.

It has now been discovered that lipid X is an acylated α-*D*-glucosamine 1-phosphate containing β-hydroxymyristoyl groups at position 2 and 3. It is believed to be a biosynthetic precursor of lipid A and it possesses a desirable ability to stimulate the activity of immune cells in a manner similar to lipid A.

It is an object of the present invention to disclose the structure of lipid X and derivatives of lipid X, the preparation of such compounds, the preparation of radiochemically labeled material, pharmaceutical compositions containing such compounds and methods of stimulating the activity of immune cells of an animal *in vitro* employing such compositions.

According to the present invention there is provided a pharmaceutical composition comprising an immunostimulating amount of the compound of the formula

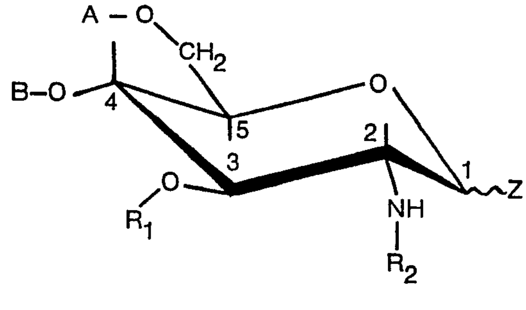

II

in which the sugar stereochemistry is that of D-glucosamine; A and B are the same or different, and are H, a hydrocarbon structure, a fatty acyl chain, or another functional group; $R_1$ represents an hydroxymyristate residue; and $R_2$ represents a hydroxymyristate residue optionally substituted with a fatty acyl chain, and when a hydroxyl substituent is present on A and/or B it may be further substituted with a fatty acyl chain; wherein a fatty acyl chain is an alkanoyl or a hydroxyalkanoyl chain of 2—24 carbons or an alkenoyl chain of 3—24 carbons; and substituent Z is a water-solubilising group;
in combination with a pharmaceutical carrier.

In a preferred composition the compound is lipid X having the formula

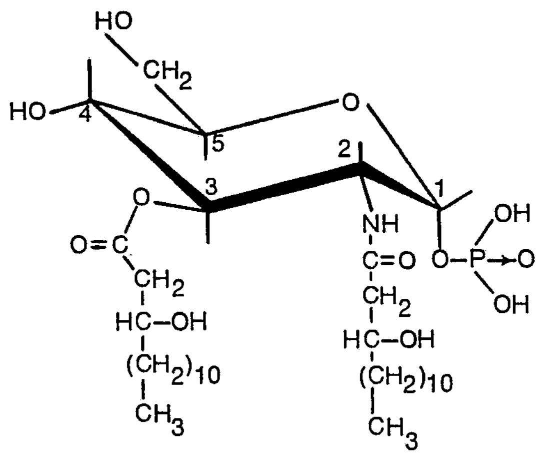

I

In another preferred composition, the compound is lipid Y having the formula

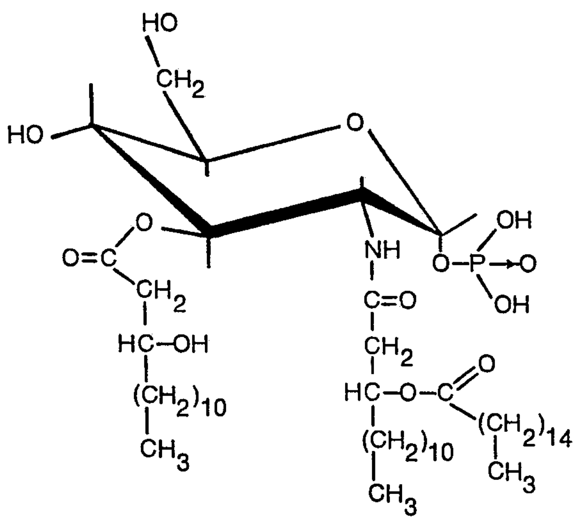

III

The present invention further provided the use of a compound represented by the formula

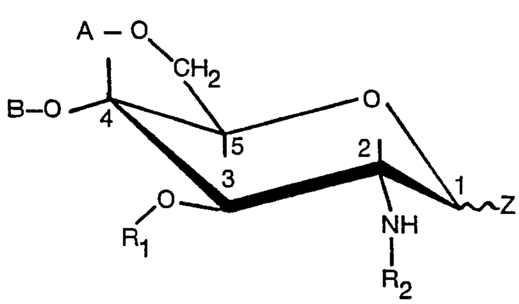

II

in which the sugar stereochemistry is that of D-glucosamine; A and B are the same or different, and are H, a hydrocarbon structure, a fatty acyl chain, or another functional group; $R_1$ represents an hydroxymyristate residue; and $R_2$ represents a hydroxymyristate residue optionally substituted with a fatty acyl chain, and when a hydroxyl substituent is present on A and/or B it may be further substituted with a fatty acyl chain; wherein a fatty acyl chain is an alkanoyl or a hydroxyalkanoyl chain of 2—24 carbons or an alkenoyl chain of 3—24 carbons; and substituent Z is a water-solubilising group in combination with a pharmaceutical carrier in the preparation of an immunostimulating agent.

In a preferred use the compound is lipid X having the formula

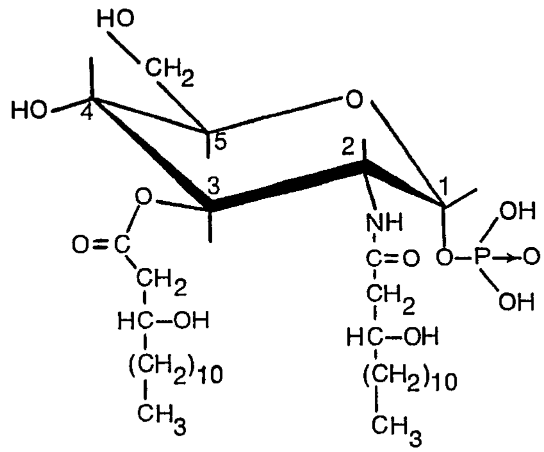

I

In another preferred use, the compound is lipid Y having the formula

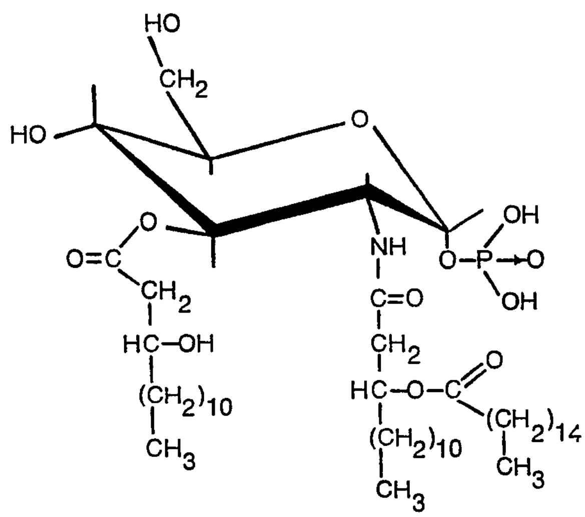

III

The present invention also provides a method of stimulating the activity of immune cells of an animal *in vitro* which comprises introducing to the immune cells of the animal a lipid X having the formula

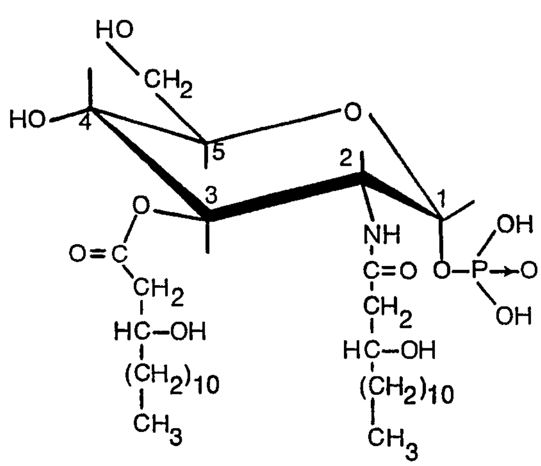

I

A method of stimulating the activity of immune cells of an animal *in vitro* which comprises introducing to the immune cells of the animal a lipid Y having the formula

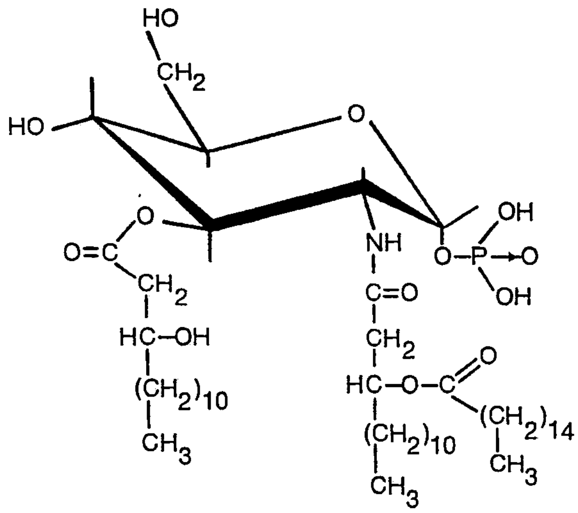

III

is also provided.

The present invention provides a compound of the formula

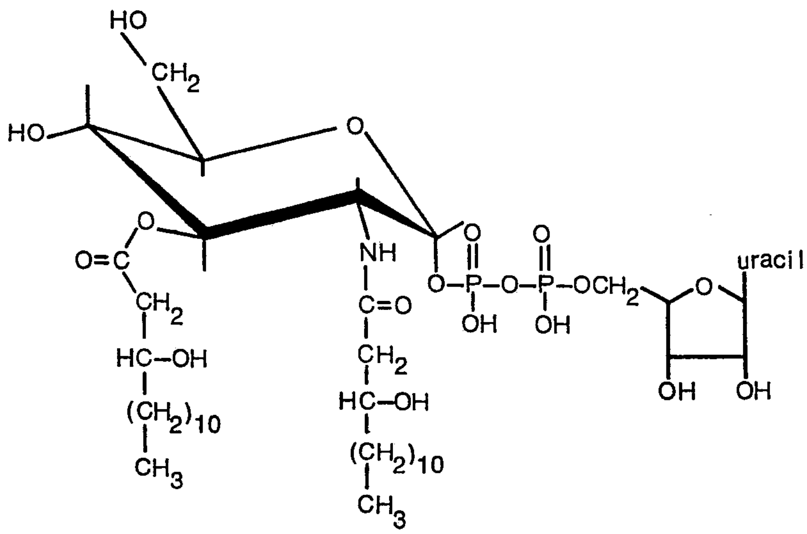

V

a compound of the formula

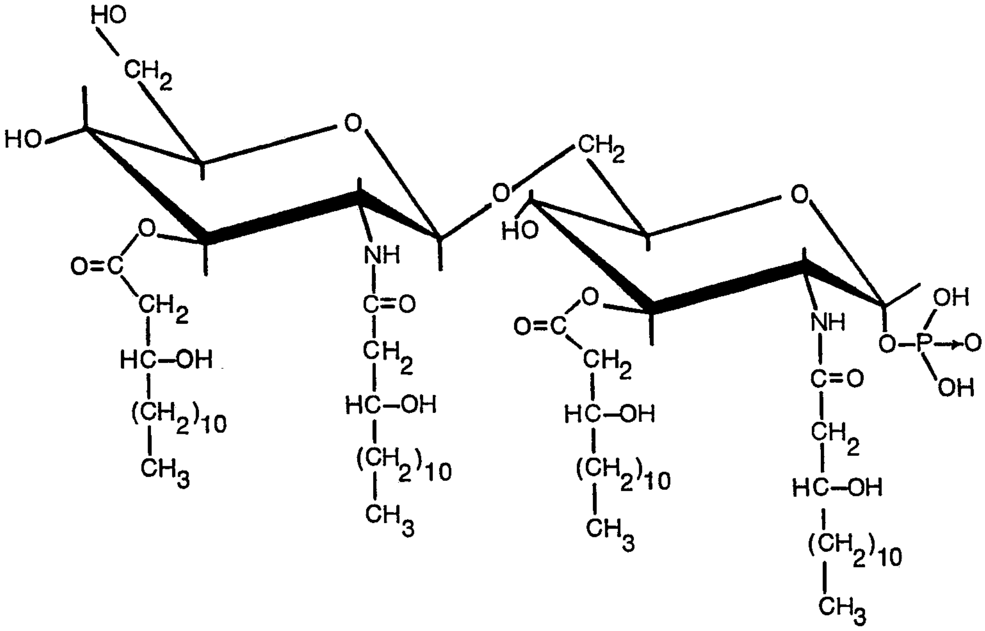

VI

which may be derived from V and I in the presence of *E. coli* enzyme preparations;

a compound of the formula

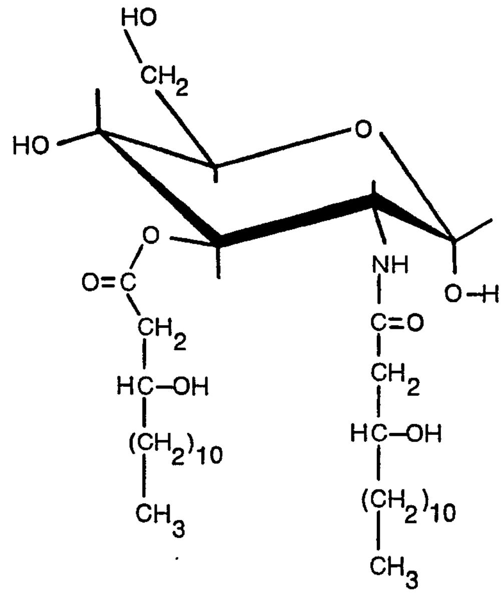

VII

and a compound of the formula

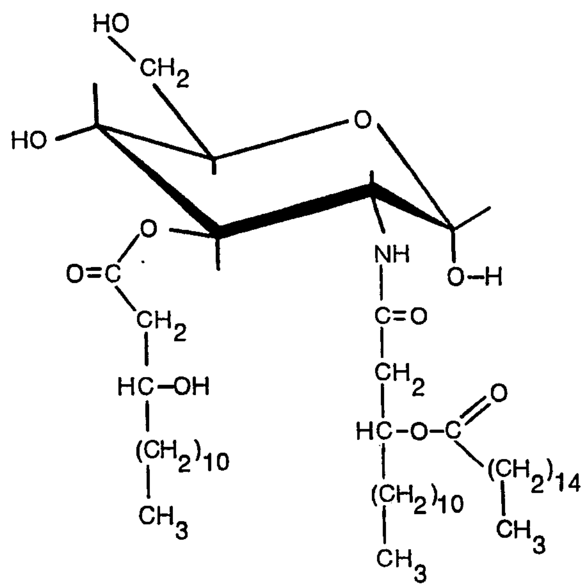

VIII

Based on fast atom bombardment mass spectrometry and proton nuclear magnetic resonance studies, we have discovered that lipid X is an acylated monosaccharide derived from glucosamine 1-phosphate. Lipid X has a $M_r$ of 711.87 as the free acid ($C_{34}H_{66}NO_{12}P$) and contains two β-hydroxymyristate moieties, one attached as an amide at the 2 position and the other as an ester at the 3 position of the sugar. It has free hydroxyl groups at the 4 and 6 positions, and the anomeric configuration is alpha. The structure of lipid X closely resembles the reducing end subunit of lipid A, and it might represent a very early precursor in the biosynthesis of lipid A.

The structure of lipid X may be represented as follows:

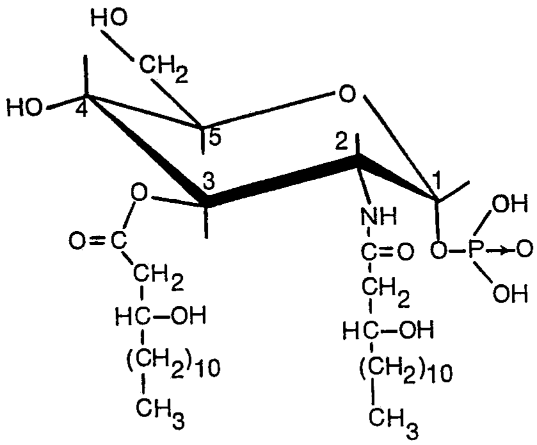

I

Lipid X and its immuno stimulating derivatives may be represented by the following general formula:

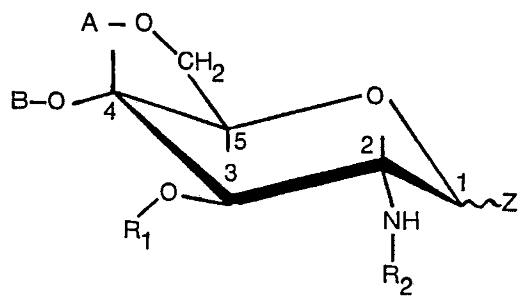

II

in which the sugar stereochemistry is that of D-glucosamine; A and B are the same or different, and are H, a hydrocarbon structure (as defined below) or a fatty acyl chain (as defined below) or another functional group (as defined below); $R_1$ represents an hydroxymyristate residue and $R_2$ represents a hydroxymyristate residue optionally substituted with a fatty acyl chain (preferably beta-hydroxymyristoyl as in the natural product (I)). When a hydroxylated substituent is present on A and/or B it may be further substituted with a fatty acyl chain. Substituent Z is a water-solubilizing group such as a hydroxyl, a phosphate, a succinate, a sulfate, a sugar residue, or a nucleotide.

A hydrocarbon structure may be an alkyl or a hydroxyalkyl group of 1—24 carbon atoms, or it may be an alkenyl group of 2—23 carbons. A fatty acyl chain is an alkanoyl or a hydroxyalkanoyl chain of 2—24 carbons, or an alkenoyl chain of 3—24 carbons. A functional group may be a sugar or a water solubilizing group, such as a succinoyl residue, a phosphate, a sulfate, or a nucleotide.

Lipid X may be chemically synthesized or it may be isolated from bacterial sources or modified by a combination of approaches.

The isolation of lipid X from a bacterial source and verification of its structure are described in the experimental work which follows:

Experimental Procedures

*Bacterial Strains and Media* — Temperature sensitive *E. coli* K12 strains MN7 (ATCC No. 39328) was grown in LB broth, which contains 10 g of NaCl, 10 g of tryptone, and 5 g of yeast extract/liter. Maximum accumulation of lipid X occurred when a log phase culture grown at 30°C to an absorbance at 550 nm of 0.4—0.6 was shifted to 42°C for 3 h. This procedure was used whether we grew small shaker cultures or large cultures (300 liters). At the time of harvest, the $A_{550nm}$ was 1.8. In a typical 300 l. fermentation, the cells were harvested by centrifugation through a continuous flow centrifuge and the cell paste was stored at −80°C. The yield was about 700 g. of cell paste per 300 l. fermentation.

Growth Conditions for Radiochemical Labelling of Lipid X

Cells of *E. coli* K12 strain MN7 were prepared by first growing them at 30°C in 200 ml of LB broth to an absorbance at 550 nm of 0.5. Then 1 mCi of [1-$^{14}$C]-acetate (60 mCi/mmol or higher) was added to the culture and incubation was continued at 42°C for 4 hr. The cells were harvested by centrifugation at 5,000 × g for 15 min. These cells were the source of the $^{14}$C labeled lipid X.

To label lipid X with $^{32}P_i$, a 50 ml culture of MN7 growing on medium was allowed to reach $A_{550}$ = 0.8 at 30°C. The cells were collected by centrifugation and resuspended, in the same volume of medium lacking phosphate. Next, the cells were incubated in shaking culture at 42°C,. $^{32}P_i$ (100 µCi/ml carrier free) was

added, and the incorporation of label was allowed to take place for 3 hours. Finally, the cells were recovered by centrifugation, and the lipid X was obtained by the rapid radiochemical extraction described below.

*Analytical and Preparative Thin Layer Chromatography (TLC)* — TLC was performed either on silica gel H or 60 using either chloroform-methanol-water-concentrated ammonium hydroxide (50:25:4:2, v/v) (solvent A) or chloroform-pyridine-formic acid (20:30:7, v/v) (solvent B).

*Extraction and Fractionation of Lipids — Method I.* Lipopolysaccharide was prepared from 110 g of cell paste by the method of Galanos et al. *Eur. J. Biochem.* 9, 245—249 (1969). The yield of crude lipopolysaccharide (including lipids X and Y) was 337 mg. Next, X and Y were extracted from this crude material with 90 ml of chloroform-methanol-water (30:10:1, v/v) to yield 93 mg of a mixture predominantly consisting of lipids X and Y. This preparation (59 mg) was subjected to preparative TLC using 20 × 20 cm silica gel H (500 μm) plates and solvent A at a load of 3 mg/plate. The bands were visualized with $I_2$ vapor and recovered by extracting the silica gel with chloroform-methanol-water (66:33:4, v/v). The yield of lipid X was 30.3 mg.

*Method II.* This method is a modification of the acidic Bligh-Dyer extraction described in *Can. J. Biochem Physiol. 37,* 911—918 (1959), which was designed for more rapid, large scale purifications. About 35 g of cell paste was suspended in 950 ml of chloroform-methanol-water (1:2:0.8, v/v), and the mixture was shaken vigorously in a 1 l Erlenmeyer for 60 min. at 30°C. The cell debris was removed by centrifugation at 5,000 × g for 10 min. To the supernatant was added 250 ml each of chloroform and water to yield a two-phase system. After further addition of 10 ml. of concentrated HCl and vigorous shaking in a 2 liter separatory funnel, the layers were allowed to separate, and the lower phase was washed once with fresh, acidic pre-equilibrated upper phase. The washed lower layer was centrifuged to break the emulsion completely, and it was concentrated by rotary evaporation. The residue was dissolved in 60 ml of chloroform-methanol-water (2:3:1, v/v) and applied to a 1.5 × 25 cm column of DEAE cellulose (acetate form). The column was successively washed with 100 ml of the same solvent and 100 ml of chloroform-methanol-40 mM ammonium acetate, pH 7.4 (2:3:1, v/v). Finally lipid X (along with lipid Y, phosphatidic acid and cardiolipin) was eluted from the column with 100 ml of chloroform-methanol-100 mM ammonium acetate, pH 7.4 (2:3:1, v/v).

The eluted material was detected by charring 5 μliter samples of each fraction, and the peak fractions were pooled (75 ml final volume). Next, enough chloroform, methanol and phosphate-buffered-saline were added in Bligh-Dyer proportions to give an upper phase volume of 500 ml (approximately 1 ml of upper phase per 100 μg of lipid X). Under these conditions lipid X partitioned into the aqueous-methanol phase, while Y and other phospholipids remained in the chloroform layer. The purified lipid X was recovered from the upper phase by adjusting the pH to 1.0 with HCl and adding a fresh organic phase. This sample was dried by rotary evaporation, redissolved in 3 ml of chloroform-pyridine-formic acid (37:30:7, v/v), and finally purified on an 0.8 × 25 cm silicic acid column equilibrated in this solvent system. The pyridine and formic acid in samples from the final silicic acid column were removed by adding methanol and water in Bligh-Dyer proportions relative to the $CHCl_3$. Additional concentrated HCl was then added until the pH of the upper phase was 1. The upper phase was removed, and the lower phase was washed twice with pre-equilibrated acidic upper phase. The washed lower phase was dried under a stream of $N_2$ to recover lipid X, presumably as the free acid. Final recovery was about 30 mg and the material was stored dessicated at −80°C.

Lipid Y was isolated essentially by the same method as X. Following DEAE cellulose chromatography and partitioning at neutral pH (see above), the lower phase containing Y and some contaminating phospholipids was dried by rotary evaporation. The residue was redissolved in 4 ml of chloroform-pyridine-formic acid (60:30:7, v/v). Final purification was achieved on a silicic acid column (0.8 × 25 cm) equilibrated with the same solvent. The pyridine and formic acid was removed as described above for lipid X. Recovery of Y in the free acid form was about 12 mg/50 gm cell paste.

Rapid Preparation of Radiochemically Labeled Lipid X

Cells of MN7 labeled with $^{32}P_i$ or $^{14}C$ acetate (as above) are extracted under Bligh-Dyer conditions but at pH 7 by use of phosphate-buffered saline as the aqueous component. In this case lipid X is recovered in the upper aqueous-methanol phase, while phospholipids and Y are in the lower phase. Relatively pure lipid X can be recovered from the upper phase by adjusting the pH to 1 with concentrated HCl and adding fresh pre-equilibrated lower phase. In this way the X is shifted back to the lower phase, where it can be recovered. Radiochemical purity by TLC in solvents A or B is about 95%.

This rapid preparation does not require column chromatography. If material of greater than 99% purity is required, this can be achieved by silicic acid chromatography in chloroform-pyridine-formic acid (37:30:7) as described above. Radiochemical preparatioon of lipid Y is not possible with the rapid technique.

*Dephosphorylation of Lipid X* — About 2—3 mg of sample was suspended by sonication in 2.0 ml of 0.1 N HCl, heated at 100°C for 15 min and cooled. Then 5 ml of chloroform-methanol (2:1, v/v) was added, mixed and allowed to stand for 10 min. The upper aqueous layer was removed, and the upper aqueous layer of the blank chloroform-methanol-water (10:5:6, v/v) mixture was used to wash the lower organic layer

containing the dephospohorylated product ("dephospho X"). The organic layer was filtered and dried with a stream of nitrogen. The extent of dephosphorylation was 80—90%.

*Preparation of Dimethyl Derivative of Lipid X* — About 2—3 mg of purified material was dissolved in 1.0 ml of chloroform-methanol (9:1, v/v) and treated with a few drops of diazomethane in diethyl ether that was sufficient to give a faint yellow color. This resulted in the methylation of the phosphate group and the formation of a phosphate triester ("dimethyl X"). After 30 min at 25°C, the solution was dried with a stream of nitrogen and dissolved in 0.3 ml of $CDCl_3$ for NMR spectroscopy.

Preparation of dephospho Y and dimethyl Y was carried out exactly as for the corresponding X derivatives.

*Preparation of UDP-diacylglucosamine and other nucleotide derivatives of lipid X.* To 5 mg of lipid X dissolved in anhydrous pyridine are added 6 mg of UMP-morpholidate. After an overnight incubation at 37°C in a tightly stoppered tube, the product is purified by preparative thin layer chromatography in solvent B, using 500 µm silica gel H plates. The nucleotide, which migrates just off the origin, is eluted and the yield is 70% of theoretical. FAB mass spectrometry of the nucleotide reveals a molecular weight of 1018, as predicted for a compound with structure V. The above method may be used to prepare any derivative of II in which Z is a nucleoside diphosphate, and may be used to make nucleotide derivatives of Y.

Reaction of UDP-diacylglucosamine (V) with lipid X (I)

Incubation of 0.2 mM V with 0.2 mM I in the presence of 1 mg per ml of a wild-type *E. coli* K12 cell-free extract in 20 mm HEPES buffer at pH 8 results in the formation of a disaccharide 1-phosphate with the structure of VI. This disaccharide can be reisolated from the reaction mixture by the methods described above for the purification of lipid Y or by preparative TLC.

*Chemical Synthesis and Modification of Lipid X and Related Compounds.* Methods for selective acylation or alkylation of sugar hydroxyl groups are known. Products of partial acylation or alkylation can be separated from each other by high performance liquid chromatography or column chromatography.

Compounds of formula II can be chemically synthesized as shown in Scheme 1. Suitable known starting materials would be the allyl 2-acylamido-3-*O*-acyl-4,6-*O*-benzylidene-2-deoxy-β-*D*-glucopyranosides.

SCHEME I

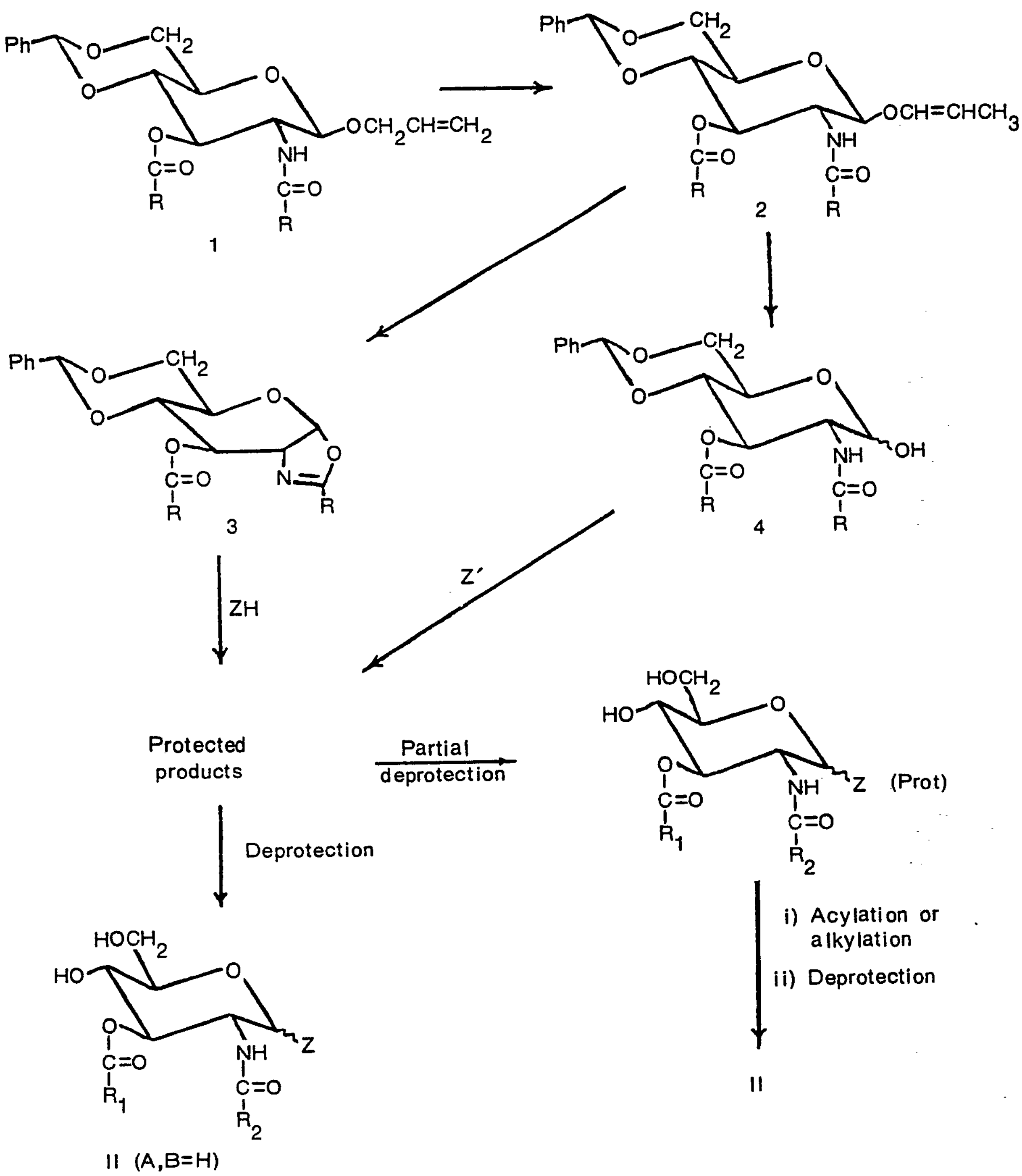


The allyl glycosides (*1*) can be isomerized to 1-propenyl glycosides (2) by treatment with tris(triphenylphosphine) rhodium(I) chloride or with another suitable isomerization catalyst. Treatment of the 1-propenyl glycosides (*2*) with mercuric chloride-mercuric oxide in an anhydrous medium will yield oxazolines (*3*). If water is included in the reaction mixture the products will be the 1-hydroxy compounds (*4*).

The reaction of the oxazolines (*3*) with precursors of Z groups, such as diesters of phosphoric acid or with alcohols or partially protected sugars in the presence of ferric chloride or other acid catalysts will result in the attachment of (protected) Z groups to position 1 of the sugar. Removal of the 4,6-*O*-benzylidene group, by hydrogenolysis or mild treatment with aqueous acid, and removal of any protecting groups from the Z-moiety, will give products of structure II, with A and B = H.

Other compounds of the series can be more expeditiously made by treating the 1-hydroxy compounds (*4*) with electrophilic reagents, designated Z′ in the scheme, such as acid anhydrides (*e.g.* succinic anhydride), acid chlorides, or phosphorochloridates in the presence of suitable bases. Deprotection will again give II (A, B = H).

If the products of the reactions of (*3*) with ZH, and of (*4*) with Z′, are treated so as to selectively remove the 4,6-*O*-benzylidene group (partial deprotection), then OH-6 and OH-4 can be alkylated or acylated, completely or selectively, by standard methods. After final deprotection, the products would have the structure II, with A and B as defined above.

*HPLC Fractionation* — High Pressure Liquid Chromatography (HPLC) was performed with two 6000A solvent delivery systems a solvent programmer, a universal liquid chromatograph injector, a variable wavelength detector and a Radial Compression Module. A 8 mm × 10 cm Radial pak A cartridge ($C_{18}$-bonded silica, 10 μm) was used. For the analysis of lipid X, a linear gradient of 0 to 100% 2-propanol-water (85:15, v/v) in acetonitrile-water (1:1, v/v) was used over a period of 60 min at a flow rate of 2 ml/min. Both solvent systems contained 5 mM tetrabutylammonium phosphate. Samples of HPLC analysis were dissolved in chloroform-methanol (4:1, v/v). The absorbance was monitored at 210 nm.

*Mass Spectral analysis* — FAB mass spectrometry was performed on a MS-50 mass spectrometer at ambient temperature, utilizing a neutral beam of xenon atoms from a saddle field discharge gun with a translational energy of 8 Kev and a discharge current of 0.4 mA. Samples (100 μg) were disolved in 100 μl of chloroform-methanol (1:1, v/v) and mxied with an equal volume of either triethanolamine (for negative mode) or monothioglycerol (for positive mode). The sample (5 μliter) was deposited on the FAB probe tip and the volatile solvents were pumped away in the insertion lock chamber of the mass spectrometer. Both negative $(M - H)^-$ and positive $(M + \text{cation})^+$ ion mass spectra were obtained by scanning at a rate of 90 atomic mass units per sec. Measurements were based on a calibration standard glycerol-potassium iodide (1:1, mol/mol) as cluster ions. Mass assignments were made with an accuracy of ±1.0 atomic mass units using the DS-55 data system.

*Proton NMR Analysis* — Spectra were recorded at 200 or 270 MHz, on Nicolet NT-200 and Bruker WH-270 Fourier transform, superconducting spectrometers interfaced with Nicolet 1280 and 1180 computers, respectively. In decoupling experiments the parent spectrum was first determined with the decoupler power set "off resonance"; then the decoupled spectrum was determined. Difference spectra were generated by subtracting the parent spectrum from the decoupled spectra.

## Results

*Analysis of Purity of Lipid X* — Purified lipid X was examined by analytical TLC using solvents A and B and found to give a single major band which could be visualized with $I_2$ vapor, by charring, or by spraying with an organic phosphate-specific molybdenum reagent. Lipid X was readily separable from the incomplete lipid A which migrated very slowly in the thin layer system with solvent A.

Chemical analysis of a sample of lipid X purified by TLC, and containing some silica, showed the following: total phosphorus, 0.96 μmol/mg; glucosamine, 1.02 μmol/mg; KDO, 0.04 μmol/mg. The phosphorus-glucosamine-KDO molar ratio was 1.00:1.06:0.04. The presence of glucosamine as the sole amino sugar was confirmed by the use of the amino acid analyzer on an acid hydrolyzed sample. These results confirmed the results of a previous chemical analysis of lipid X.

Purified lipid X was analyzed by reverse-phase HPLC before and after preparative TLC fractionation (Method I). This analysis showed a single peak of lipid X eluting at 30 min with an estimated purity by peak height analysis of at least 95%. There was no evidence for the presence of a homologous series or further microheterogeneity of lipid X. Under these conditions of HPLC, a purified monophosphoryl lipid A designated TLC-3 from *S. typhimurium* and containing 6 fatty acid residues came off the column at the end of the gradient.

*Nature of the Phosphate Group in Lipid X* — When [$^{14}$C]lipid X was treated with 0.1 N HCl at 100°C for 15 min, a new product was formed with a $R_f$ value of 0.53 on TLC in solvent A (unhydrolyzed lipid X had $R_f$ 0.12). The radioactivity pattern of the TLC separation showed that almost all of the label in [$^{14}$C]lipid X was shifted to the new product after hydrolysis. The time-course of acid-catalyzed hydrolysis of [$^{14}$C]lipid X was recorded. Over a period of 15 min, there is a rapid decrease of [$^{14}$C]lipid X and a corresponding rise in the level of new product.

The distribution of the phosphorus content in the aqueous and organic phases of lipid X before and after 15 min hydrolysis is shown in Table I.

TABLE I

Distribution of phosphate in the aqueous and organic phase of control and acid-hydrolyzed lipid X. The sample was prepared as described. Then 1/3.25 volume of the aqueous phases and 1/3.75 volume of the organic phases were analyzed for phosphate content.

| | nmol phosphate | |
|---|---|---|
| Two-phase system | Control | Acid hydrolyzed |
| Aqueous layer[a] | 48.4 | 495.3 |
| Organic layer | 564.8 | 97.5 |

[a]Inorganic phosophate assay (digestion was omitted).

The phosphate content is shifted from the organic phase before hydrolysis to the aqueous phase after hydrolysis. By utilizing the data in Table I and establishing the partition coefficient of lipid X in the chloroform-methanol-water (20:10:9, v/v) system, the extent of conversion after 15 min hydrolysis was calculated to be 78%. Quantitative kinetic analysis of the hydrolysis reaction is complicated by the tendency of lipid X to aggregate in aqueous solutions, and by the precipitation of the product (dephospho X). A logarithmic plot of the time-course data reveals a modest decrease of the hydrolysis rate constant with time. The evidence supports the conclusion that lipid X contains a single type of acid-labile phosphate, attached to the 1-position of the glucosamine.

The results of negative and positive mass spectrometry established the precise value of $M_r$ for lipid X to be 711.87 as the free acid ($C_{34}H_{66}NO_{12}P$). A molecule of lipid X contains one glucosamine, two hydroxymyristate residues, and one phosphate, the latter linked to position 1 of the sugar. Since one of the hydroxymyristic linkages is stable to mild alkali and the other is cleaved by mild alkaline treatment, one hydroxymyristate must be amide-linked (at position 2 of the glucosamine) and the other ester-linked.

*Proton NMR Analysis of Lipid X* — Four possible sites for the ester-linked hydroxymyristate had to be considered, namely positions 3, 4 and 6 of the glucosamine residue, and β-position of the amide-linked hydroxymyristoyl group. The principal purpose of the NMR spectroscopic analysis was to distinguish between these alternatives. Initially, spectra was taken of the free acid form of lipid X dissolved in $CDCl_3$, but these were poorly resolved, presumably because of the aggregation of the amphipathic lipid in solution. To obtain satisfactory resolution it was necessary to esterify the phosphate moiety with diazomethane, or remove it, and dissolve the resulting derivatives (dimethyl X and dephospho X, respectively) in $CdCl_3$ with 1—10 percent dimethyl sulfoxide-$d_6$.

The spectrum of dephospho X (not shown) resembled that of dimethyl X. Data from decoupling experiments on dephospho X substantiated the assignments made from experiments with dimethyl X.

The normal range of the resonances for H-3 and H-4, and the downshifting of these resonances on acylation at the respective positions, is well established by observations on numerous glucosamine derivatives, including synthetic lipid A analogs. Thus, the downfield position of the H-3 signal in the present case clearly delineates O-3 of the glucosamine as the site of attachment of the ester-linked hydroxymyristate in lipid X.

## Discussion

The complete structure of the glycolipid, lipid X, which accumulates in certain phosphatidylglycerol-deficient mutants of *E. coli* (particularly at nonpermissive temperature) has now been established. Lipid X is 2-deoxy-2-[(R)-3-hydroxytetradecanamido]-3-*O*-[(R)-3-hydroxytetradecanoyl]-α-*D*-glucopyranose 1-phosphate and it bears a striking resemblance to the reducing end subunit of lipid A. The structure of lipid Y, which is essentially the same as X but contains an additional esterified palmitoyl residue on the Beta-OH of the N-linked hydroxymyristate, has also been completed using techniques essentially identical to those described above for X.

Lipid X might be a very early precursor of lipid A biosynthesis. Thus radiolabeled lipid X could be useful as a substrate to study the pathway for the enzymatic synthesis of lipid A in a cell-free system.

Lipid X might serve as a good model compound to study the relationship between the structure of lipid A and its numerous biological activities. The attractive feature of lipid X is the simplicity of its structure and the ease with which the structure can be modified by controlled degradation. Preliminary experiments show that lipid X is relatively nontoxic in the chick embryo lethality test and that it is a B-cell mitogen.

There appears to be an interaction between phosphatidylglycerol metabolism and lipid A biosynthesis. Mutants like 11—2 or MN7 (*pgsA444 pgsB1*) are isolated by a two stage mutagenesis. They are

temperature-sensitive for growth and phosphatidylglycerol-deficient only when both genetic lesions are present. Based on subcloning of *pgsA* and detailed enzymological studies, it is certain that *pgsA* represents the structural gene for phosphatidylglycerolphosphate synthase. The *pgsB* may code for any enzyme in the lipid A pathway, for instance one that converts lipid X to the next intermediate. In this regard, it is relevant that strains with the genotype *pgsA*$^+$ *pgsB1* regain normal phosphatidylglycerol levels and are not temperature-sensitive, but they continue to have more than 100 times the lipid X present in wild-type cells (*pgsA*$^+$ *pgsB*$^+$). Perhaps, the accumulation of lipid X caused by the *pgsB1* mutation interferes with phosphatidylglycerolphosphate production when the synthase specified by the *pgsA444* allele is present. Furthermore, phosphatidylglycerol itself might be required for lipid X utilization, since all components of phospholipid metabolism and lipid A biosynthesis are membrane-bound.

The Biological Acitivity of Lipid X

The outer membrane of gram-negative bacteria such as *Escherichia coli* and *Salmonella typhimurium* consists of proteins, phospholipids and lipopolysaccharide. The latter substance is localized almost exculsively on the outer surface of the outer membrane. It accounts for many of the immunological and endotoxic properties of gram-negative organisms. Although the complete structure of lipopolysaccharide is unknown, it consists of three domains. The outer sugars, which are highly variable, give rise to the O-antigenic determinants. The core sugars are relatively conserved and may be involved in cell penetration by certain bacteriophages. The lipid A molecule (which is highly conserved between species) functions as a hydrophobic anchor holding lipopolysaccharide in place. Since lipopolysaccharide represents several percent of the dry weight of gram-negative bacteria, it follows that lipid A (and not phospholipid) must account for most of the outer leaflet of the outer membrane.

Since lipid X is easy to purify and can be further manipulated by controlled chemical degradation, we have examined its ability to activate mouse lymphocytes. We have found that our lipid X preparations are almost as active as intact lipopolysaccharide by this criterion, and that the ester-linked hydroxymyristate at position 3 is crucial for this biological function. The results suggest that lipid X, like lipid A, is a B cell mitogen.

Methods for Evaluating Mitogenic Activity of Lipid X

Lipid X was isolated from strain MN7. The mild alkaline hydrolysis product of lipid X (which retains the *N*-linked but not the *O*-linked hydroxymyristate residue) was obtained under standard conditions for deacylation of phospholipids. Dephosphorylated lipid X was generated by mold acid treatment (0.1 M HCl at 100°C for 60 min). A lipid A derivative lacking the 1-phosphate moiety at the reducing end was generated as described in the literature. Microspheres (polystyrenedivinylbenzene beads, 5.7 $\pm$ 1.5 µM diameter, were coated with lipopolysaccharide or dephospho-X by known methods.

Mitogenesis Experiments

C57B1/10 and C3H/HeJ mice were anesthetized with ether, and the spleens were excised immediately. After opening the splenic capsule, the cells were suspended in 10 ml of DMEM, washed twice with the same, and then resuspended at 5 $\times$ $10^6$/ml in DMEM, supplemented with 10% fetal bovine serum, 2 mM L-glutamine, 1 mM sodium pyruvate, 10 mM MEM non-essential amino acids, 10 mM Hepes, 50 µM 2-mercaptoethanol, 100 units/ml penicillin G and 100 µg/ml streptomycin. Multiple wells of a 96 well Costar microtiter dish was seeded with 0.1 ml of this cell suspension (5 $\times$ $10^5$/well). Next, an additional 0.1 ml of DMEM supplemented with an appropriate amount of test mitogen was added. Following addition of mitogen, the dishses were incubated for 2 days at 37°C, 100% humidity and 7.5% $CO_2$. Finally, [*methyl*-$^3$H]-thymidine was added at 1 µCi/well, and the cells were incubated at 37°C for an additional six hours. Cells were washed free of medium and excess thymidine with 0.9% NaCl using a Bellco microharvester. The washed cells were retained on glass fiber filter strips, and radioactivity incorporated into the cells was quantitated by liquid scintillation counting. Each mitogen concentration was tested in triplicate wells, and the standard deviation of the measurement was approximately $\pm$10%. Induction of plaque forming cells by various mitogen preparations was quantitated using a monolayer of sheep red blood cells as the indicator.

Results

*Mitogenic effect of the chloroform-soluble fraction from mutant MN7.* Membrane phospholipids were extracted under acidic conditions from a strain of *E. coli* which is wild-type with respect to its membrane lipids or from mutant MN7 which accumulates lipid X. The crude lipids were dried under $N_2$ to remove $CHCl_3$, suspended in 1 mM EDTA adjustd to pH 6 with NaOH, and dispersed at a concentration of 1—2 mg/ml by sonic irradiation for 5 min at 25°C in bath sonicator. *E. coli* lipopolysaccharide was dissolved in 1 mM EDTA in a similar manner. Next, triplicate sets of mouse lymphocyte cultures were incubated for 48 hours with increasing amounts of added phospholipid (0—5 µg/ml final concentration). Following this, the cells were labeled for 6 hours with [*methyl*-3H]-thymidine, and incorporation of $^3$H into DNA was determined.

The phospholipid dispersions derived from MN7 stimulated lymphocyte proliferation to a much greater extent than similar preparations from strains of *E. coli* with a wild-type lipid composition. The stimulation of cells by commercial *E. coli* lipopolysaccharide was compared. The results suggest that there is an additional mitogenic factor in the $CHCl_3$-soluble fraction of strain MN7 that is not present in the wild-type. The main difference between MN7 and wild-type *E. coli* lipids has been attributed to the presence of glycolipids X and Y in the mutant at levels that are 10,000-fold (or more) greater than normal.

Mitogenic Activity of Purified Lipid X

The predominant glycolipid that accumulates in MN7 is lipid X, a diacylglucosamine 1-phosphate, substituted with β-hydroxymyristate at positions 2 and 3. Lipid X is readily dispersed in 1 mM EDTA (pH 6) at concentrations of 1—2 mg/ml, especially with mild ultrasonic irradiation.

Dispersions of lipid X (tested in the range of 0—50 μg/ml final concentration) are almost as effective as commercial lipopolysaccharide in stimulating lymphocyte proliferation. However, unlike the commercial lipopolysaccharide, this activity can now be attributed directly to a highly purified and structurally defined molecule. Phosphatidic acid, which is structurally similar to lipid X, has no mitogenic activity. Phosphatidylcholine, sphingomyelin, phosphatidylinositol, cardiolipin, phosphatidylserine, CDP-diglyceride, lysophosphatidylcholine, lysophosphatidylethanolamine and lysophosphatidic acid are also ineffective. Polymyxin B (50 μm/ml), which inhibits lipopolysaccharide-induced mitogenesis, also abolishes lymphocyte stimulation by lipid X. This supports the notion that lipids A and X have similar mechanisms of action and that the stimulation of the B lymphocytes is involved.

To further demonstrate that lipid X exerts its effects by the same mechanism(s) as lipopolysaccharide (or lipid A), we examined splenic lymphocytes from C3H/HeJ mice. These are unresponsive to lipopolysaccharide, presumably because they lack a membrane receptor (or enzyme) that recognizes this molecule. The C3H/HeJ lymphocytes also respond poorly (if at all) to lipid X. However, they are readily activated by the T cell mitogen concanavalin A, or by PPD-tuberculin, which function by different mechanisms. T cells do not seem to be required for lipid X mitogenesis, since splenic lymphocytes from nude athymic mice respond well to lipid X and lipopolysaccharide, but not to concanavalin A.

Molecular Requirements for Mitogenicity

Mild alkaline hydrolysis of lipopolysaccharide abolishes its mitogenic activity. Since the locations of the ester-linked fatty acids in lipid A are not precisely known, it is unclear which particular ester-linkage is required. The very fact that lipid X has strong mitogenic activity implies that the β-hydroxymyristate esterified at the 3 position of this molecule must mediate this function. To prove this, we subjected lipid X to mild alkaline hydrolysis and isolated a monoacyl glucosamine 1-phosphate derivative, bearing only the amide-linked β-hydroxymyristate. The removal of the esterified hydroxymyristate completely abolishes the B cell proliferation. Simultaneous addition of equimolar β-hydroxymyristate and monoacyl glucosamine 1-phosphate or of glucosamine 1-phosphate plus β-hydroxymyristate did not cause significant proliferation.

In addition to lipid X, mutant MN7 accumulates lipid Y, especially after 3 hours at 42°C. This material is similar to X but has an additional esterified palmitate moiety on the β-hydroxyl group of the N-linked hydroxymyristate.

Lipid Y is much less soluble in $H_2O$ than lipid X, but can still be dispersed at 1 mg/ml by sonic irradiation at pH 6. The material is also mitogenic, though somewhat less under these conditions, possibly because of poor solubility.

Mild acid treatment can be used to remove the 1-phosphate moiety from lipid X, leaving the two fatty acid residues in place. The resulting substance, termed dephospho lipid X, is very insoluble in $H_2O$ and cannot be dispersed by sonic irradiation. Fine suspension of dephospho lipid X or preparations immobilized on hydrophobic beads do cause slight cell proliferation (not shown). The finding that dephospho lipid X retains significant biological activity strongly suggests that the sugar 1-phosphate moiety is not obligatory for the mitogenic response. This conclusion is further supported by the observation that TLC-3, a lipid A derivative from *S. typhimurium* G30/C21 lacking the l-phosphate residue, also is fully mitogenic when tested under the same conditions.

Formation of Plaque-Forming Cells

We evaluated the stimulation of plaque-forming cells by *E. coli* lipopolysaccharide, lipid X, or other preparations. All derivatives which stimulated radioactive thymidine incorporation also increased the incidence of antibody-producing cells significantly. These results show that lipid X, lipid Y, and perhaps also dephospho lipid X all stimulate true lymphocyte proliferation and that the observed increase in [*methyl*-$^3$H]thymidine incorporation is not a radiochemical artifact.

Since the complete covalent structure of lipid A is not known, it has been difficult to elucidate the molecular mechanisms by which lipid A (or lipopolysaccharide) triggers diverse physiological responses, such as B cell proliferation or endotoxic shock. The discovery of biologically active monosaccharide lipid A fragments with defined structures makes it possible for the first time to explore lipid A function at a biochemical level. The lipid X molecule retains most of the properties of the intact lipopolysaccharide with respect to the induction of B lymphocyte proliferation. In this case the ester-linked β-hydroxymyristate at position 3 of the glucosamine ring is critical for function, while the phosphate moiety may enhance biological activity by increasing the solubility of the acylated sugar.

Lipid X also possesses other activities normally associated with lipopolysaccharide. Recently we have found that sheep respond to intravenous injection of lipid X in a manner that resembles the pathophysiological effects of Gram-negative endotoxin including both the characteristic pulmonary

hyptertension and the increased lung lymph flow. Further, the limulus lysate assay for Gram-negative endotoxin is positive with lipid X under certain conditions, and removal of the ester-linked hydroxymyristate abolishes clot-forming activity. On the other hand, lipid X is relatively nontoxic as judged by the chick embryo lethality test ($CELD_{50}$ >10 µg). It appears that many of the biological activities of lipid A are mediated by the esterified hydroxymyristatoyl group at position 3 of the sugar.

The compound lipid X and its immunostimulating derivatives may be introduced to the immune cells of an animal in place of lipid A to stimulate the immune cells when such stimulation is desired. When thus employed the compound(s) may be administered in the form of parenteral solutions containing the selected immunostimulating compound in the sterile liquid suitable for intravenous administration. The exact dosage of the active compound to be administered will vary with the size and weight of the animal and the desired immunological response. Generally speaking, the amount administered will be less than that which will produce an unacceptable endotoxic response in the animal.

Although specific microorganisms that produce lipid X in recoverable amounts have been described, it will be apparent that other microorganisms, including those modified by genetic engineering, may be used to prepare lipid X. It will also be apparent that lipid X and some of its useful derivatives may be chemically synthesized using conventional techniques.

**Claims**

1. A pharmaceutical composition comprising an immunostimulating amount of the compound of the formula

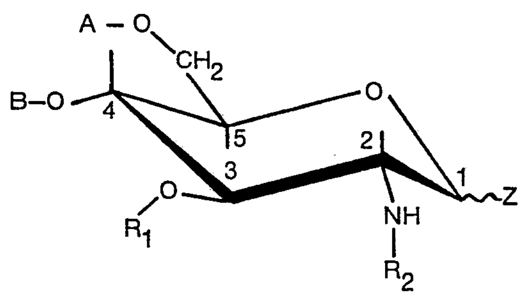

II

in which the sugar stereochemistry is that of D-glucosamine; A and B are the same or different, and are H, a hydrocarbon structure, a fatty acyl chain, or another functional group; $R_1$ represents an hydroxymyristate residue; and $R_2$ represents a hydroxymyristate residue optionally substituted with a fatty acyl chain; and when a hydroxyl substituent is present on A and/or B it may be further substituted with a fatty acyl chain;

wherein a fatty acyl chain is an alkanoyl or a hydroxyalkanoyl chain of 2—24 carbons or an alkenoyl chain of 3—24 carbons; and substituent Z is a water-solubilising group;
in combination with a pharmaceutical carrier.

2. A pharmaceutical composition according to claim 1 characterised in that the compound is lipid X having the formula

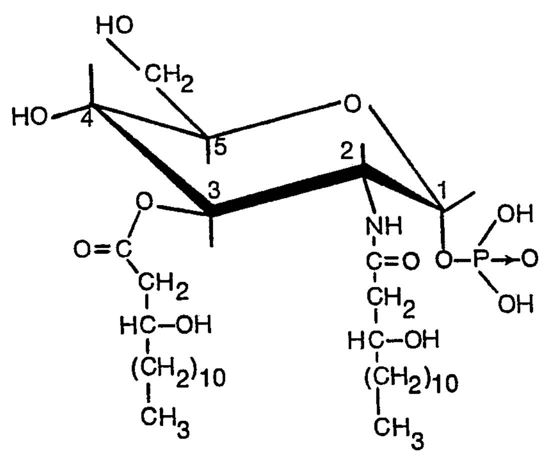

I

3. A pharmaceutical composition according to claim 1 characterised in that the compound is lipid Y having the formula

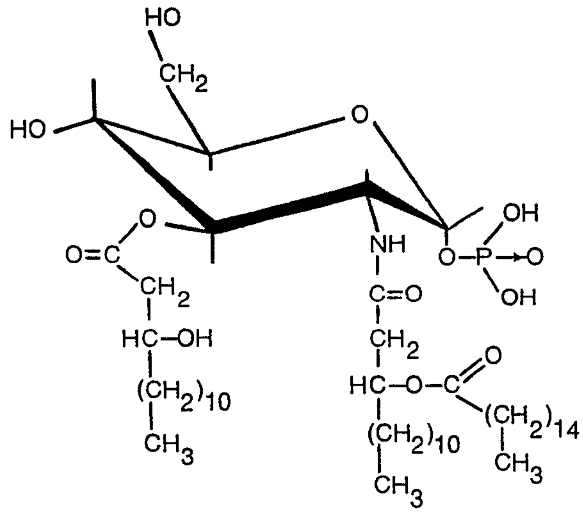

III

4. Use of a compund represented by the formula

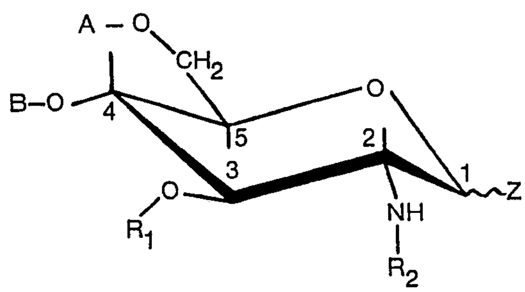

II

in which the sugar stereochemistry is that of D-glucosamine; A and B are the same or different, and are H, a hydrocarbon structure, a fatty acyl chain, or another functional group; $R_1$ represents an hydroxymyristate residue; and $R_2$ represents a hydroxymyristate residue optionally substituted with a fatty acyl chain; and when a hydroxyl substituent is present on A and/or B it may be further substituted with a fatty acyl chain, wherein a fatty acyl chain is an alkanoyl or hydroxyalkanoyl chain of 2—24 carbons or an alkenoyl chain of 3—24 carbons; and substituent Z is a water-solubilising group in combination with a pharmaceutical carrier in the preparation of an immunostimulating agent.

5. The use according to claim 4 characterised in that the compound is lipid X having the formula

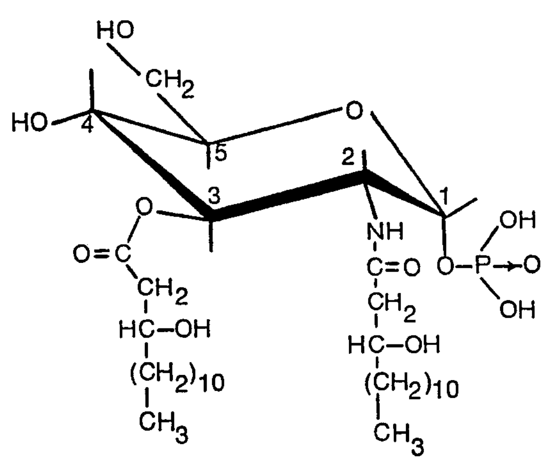

I

6. The use according to claim 4 characterised in that the compound is lipid Y having the formula

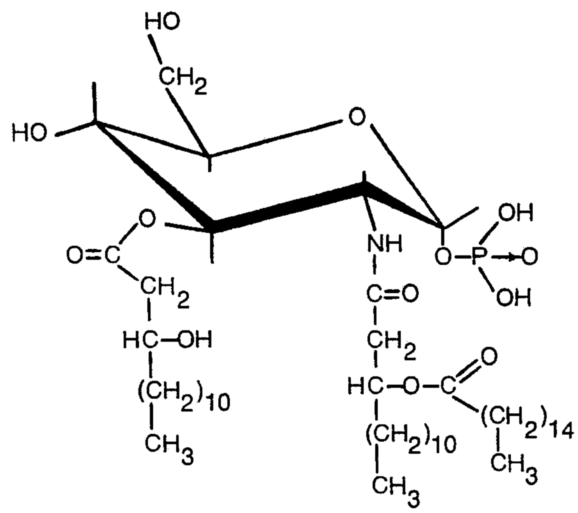

III

7. A method of stimulating the activity of immune cells of an animal *in vitro* which comprises introducing to the immune cells of the animal a lipid X having the formula

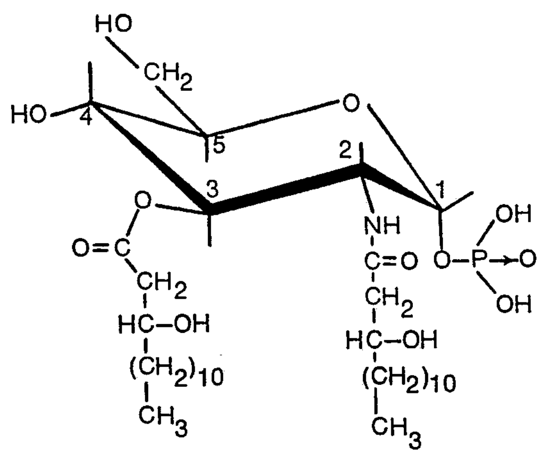

I

8. A method of stimulating the activity of immune cells of an animal *in vitro* which comprises introducing to the immune cells of the animal a lipid Y having the formula

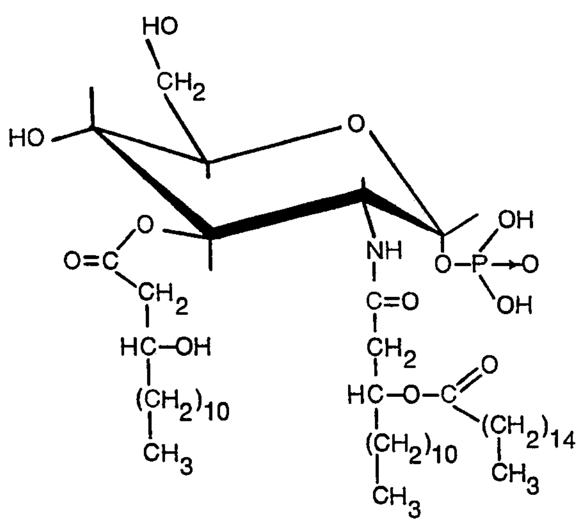

III

9. A compound of the formula

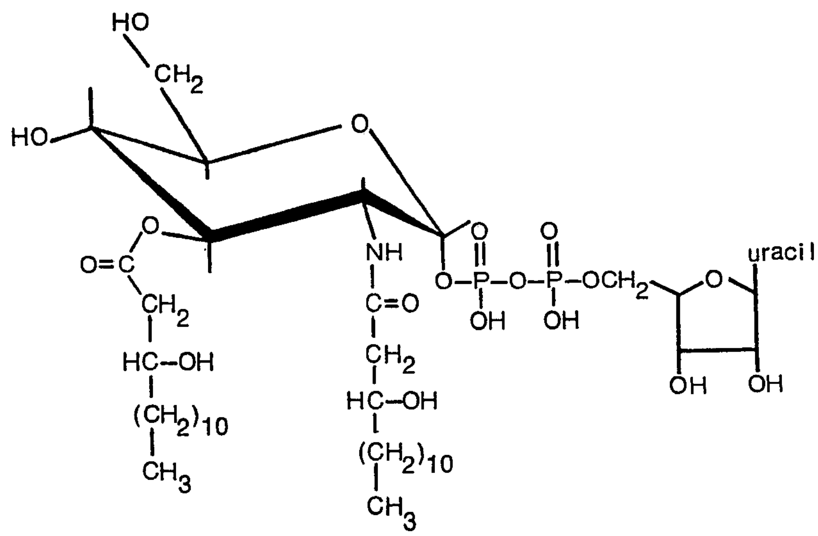

V

10. A compound of the formula

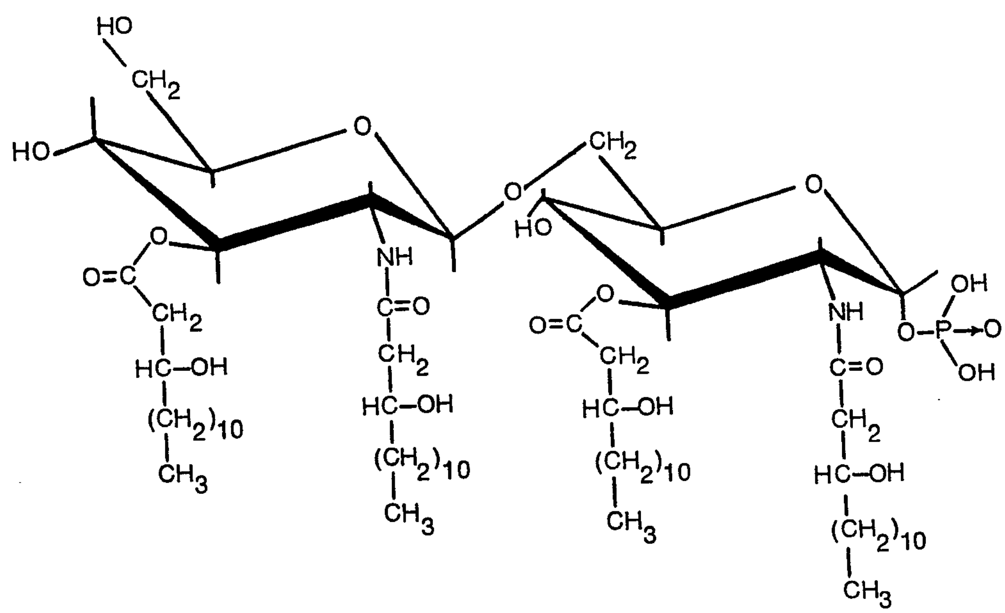

VI

which may be derived from V and I in the presence of *E. coli* enzyme preparations.

11. A compound of the formula

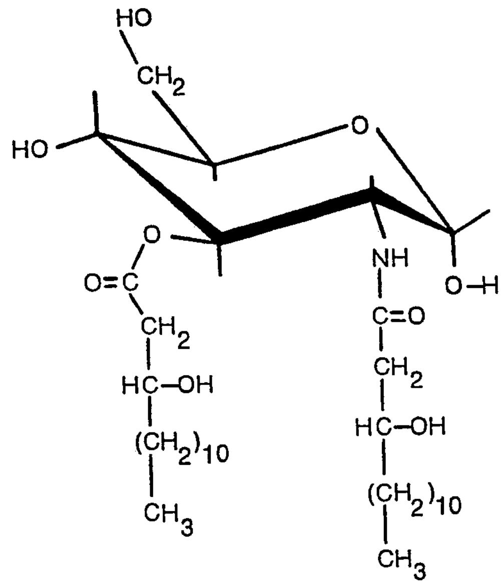

VII

12. A compound of the formula

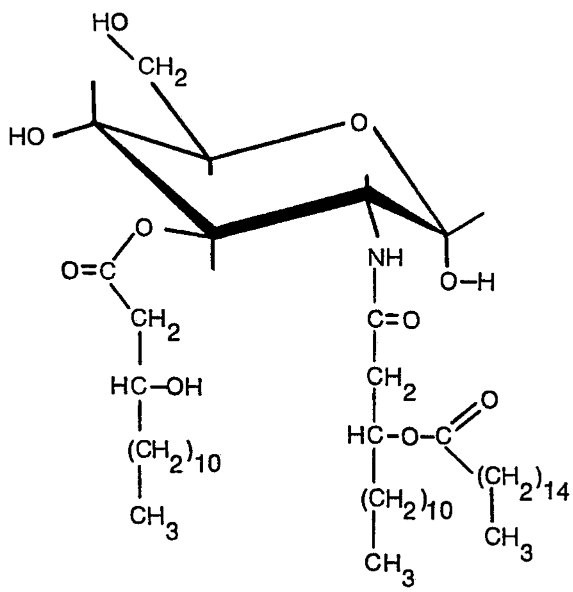

VIII

**Patentansprüche**

1. Pharmazeutische Zusammensetzung, die eine immunstimulierende Menge einer Verbindung mit der Formel

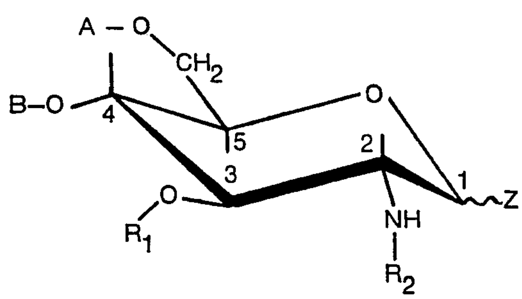

II

enthält, deren sterische Konfiguration der von D-Glucosamin entspricht und bei der A und B gleich oder unterschiedlich und H, eine Kohlenwasserstoffgruppe, eine Fettsäureacylkette oder eine andere funktionelle Gruppe sind, $R_1$ ein Hydroxymyristatrest, und $R_2$ ein Hydroxymyristatrest ist, der gegebenenfalls mit einer Fettsäureacylkette substituiert ist und bei der, wenn bei A und/oder B ein Hydroxylsubstituent vorliegt, dieser ferner mit einer Fettsäureacylkette substituiert sein kann,

wobei eine Fettsäureacylkette eine Alkanoyl- oder eine Hydroxyalkanoylkette mit 2 bis 24 Kohlenstoffatomen oder eine Alkenoylkette mit 3 bis 24 Kohlenstoffatomen, der Substituent Z eine in Wasser lösliche Gruppe ist und die Verbindung in einer pharmazeutischen Trägersubstanz vorliegt.

2. Pharmazeutische Zusammensetzung gemäß Anspruch 1, dadurch gekennzeichnet, daß die Verbindung Lipid X mit der folgenden Formel ist:

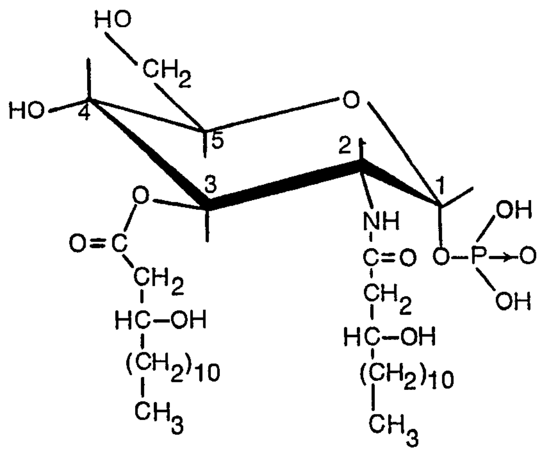

I

3. Pharmazeutische Zusammensetzung gemäß Anspruch 1, dadurch gekennzeichnet, daß die Verbindung Lipid Y mit der folgenden Formel ist:

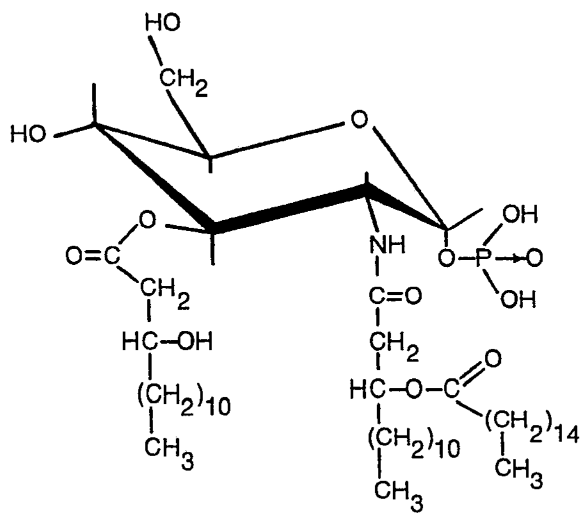

III

4. Verwendung einer Verbindung mit der Formel

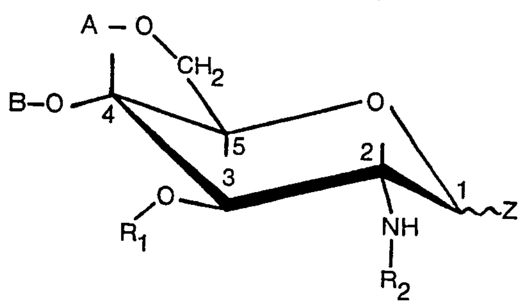

II

deren sterische Konfiguration die von D-Glucosamin ist und bei der A und B gleich oder unterschiedlich und H, eine Kohlenwasserstoffgruppe, eine Fettsäureacylkette oder eine andere funktionelle Gruppe sind, $R_1$ ein Hydroxymyristatrest, und $R_2$ ein Hydroxymyristatrest ist, der gegebenenfalls mit einer Fettsäureacylkette substituiert ist und bei der, wenn bei A und/oder B ein Hydroxylsubstituent vorliegt, dieser ferner mit einer Fettsäureacylkette substituiert sein kann,

wobei eine Fettsäureacylkette eine Alkanoyl- oder eine Hydroxyalkanoylkette mit 2 bis 24 Kohlenstoffatomen oder eine Alkenoylkette mit 3 bis 24 Kohlenstoffatomen, der Substituent Z eine in Wasser lösliche Gruppe ist und die Verbindung in einer pharmazeutischen Trägersubstanz vorliegt.

5. Verwendung gemäß Anspruch 4, dadurch gekennzeichnet, daß die Verbindung Lipid X mit der folgenden Formel ist:

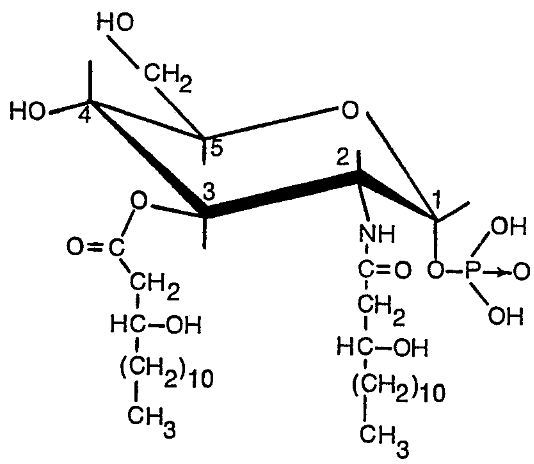

I

6. Verwendung gemäß Anspruch 4, dadurch gekennzeichnet, daß die Verbindung Lipid Y mit der folgenden Formel ist:

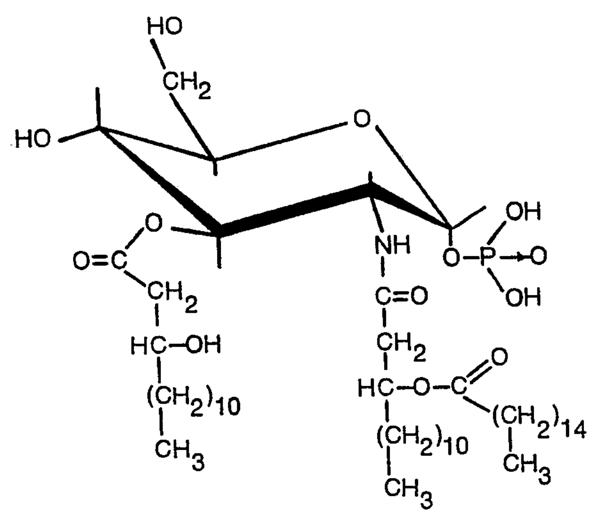

III

7. Verfahren zur Stimulation der Aktivität von immunen Zellen eines Tieres in vitro, bei dem in die immunen Zellen des Tieres ein Lipid X mit der folgenden Formel eingeführt wird:

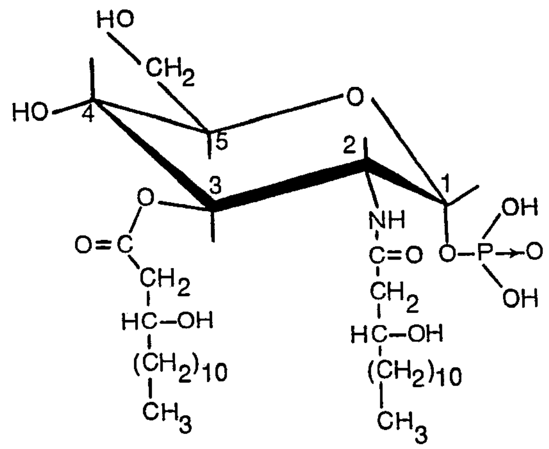

I

8. Verfahren zur Stimulation der Aktivität von immunen Zellen eines Tieres in vitro, bei dem in die immunen Zellen des Tieres ein Lipid Y mit der folgenden Formel eingeführt wird:

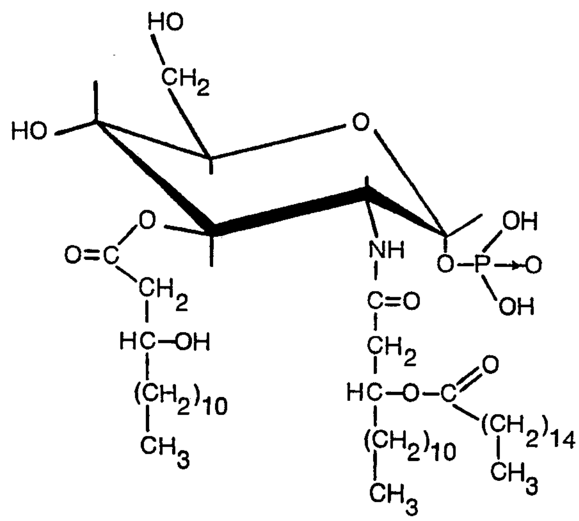

III

9. Verbindung mit der Formel

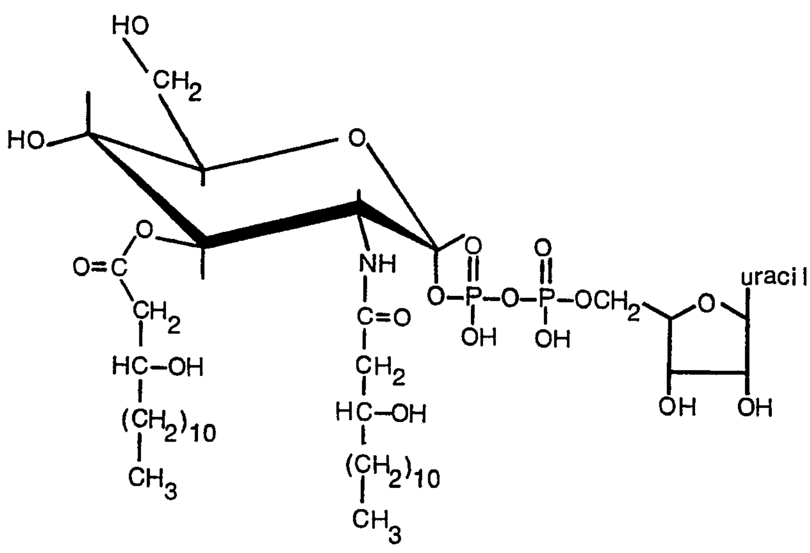

V

10. Verbindung mit der Formel

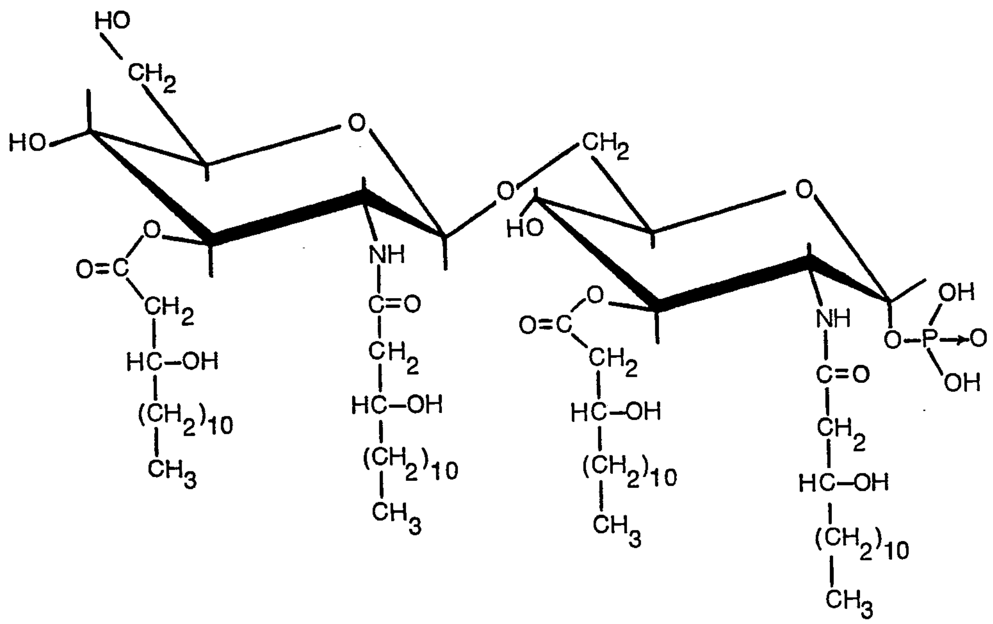

VI

die aus den Verbindungen mit der Formel V und I in Anwesenheit von *E. coli* Enzympräparaten erhalten werden können.

11. Verbindung mit der Formel

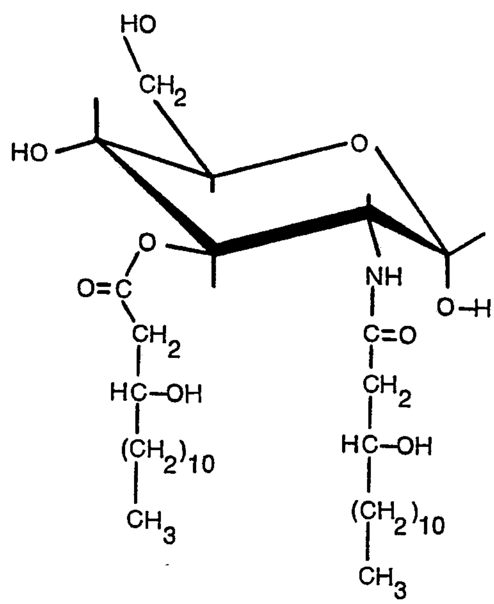

VII

12. Verbindung mit der Formel

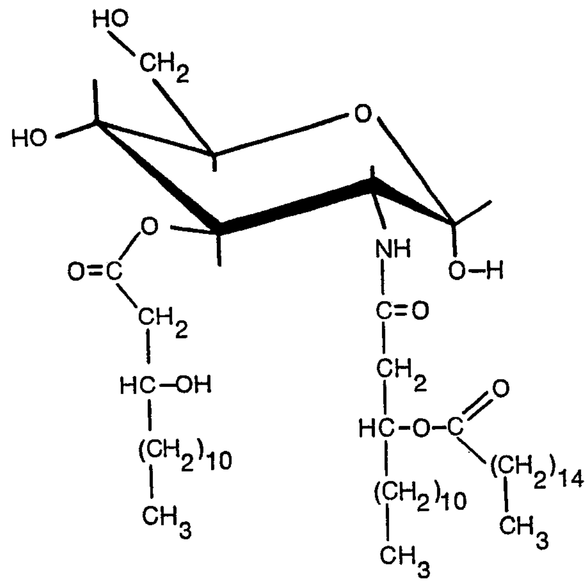

VIII

**Revendications**

1. Composition pharmaceutique comprenant une quantité immunostimulante d'un composé de formule:

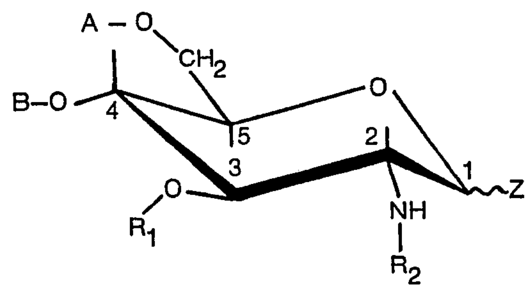

II

dans laquelle: la stéréochimie du sucre est celle de la D-glucosamine; A et B sont identiques ou différents, et représentent H, une structure hydrocarbonée, une chaîne acyle grasse, ou un autre groupe fonctionnel; $R_1$ représente un reste hydroxymyristate; et $R_2$ représente une reste hydroxymyristate facultativement substitué par une chaîne acyle grasse; et lorsqu'un substituant hydroxyle est présent sur A et/ou B, il peut être encore substitué par une chaîne acyle grasse;

où une chaîne acyle grasse est une chaîne alcanoyle ou hydroxyalcanoyle de 2—24 carbones ou une chaîne alcénoyle de 3—24 carbones et le substituant Z est un groupe de solubilisation dans l'eau; en combinaison avec un support pharmaceutique.

2. Composition pharmaceutique selon la revendication 1, caractérisé par le fait que le composé est le lipide X de formule:

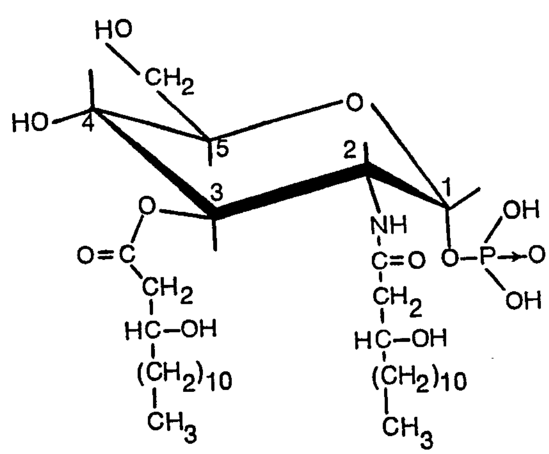

I

3. Composition pharmaceutique selon la revendication 1, caractérisé par le fait que le composé est le lipide Y de formule:

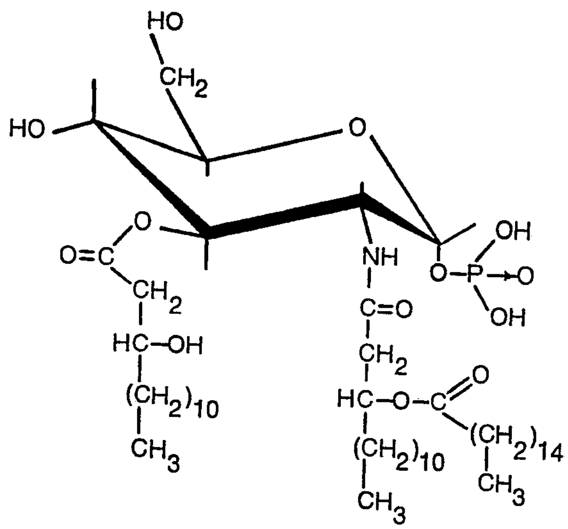

III

4. Utilisation d'un composé représenté par la formule:

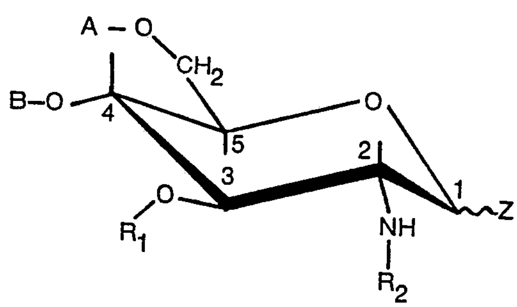

II

dans laquelle: la stéréochimie du sucre est celle de la D-glucosamine; A et B sont identiques ou différents, et représentent H, une structure hydrocarbonée, une chaîne acyle grasse, ou un autre groupe fonctionnel; $R_1$ représente un reste hydroxymyristate; et $R_2$ représente une reste hydroxymyristate facultativement substitué par une chaîne acyle grasse; et lorsqu'un substituant hydroxyle est présent sur A et/ou B, il peut être encore substitué par une chaîne acyle grasse;

où une chaîne acyle grasse est une chaîne alcanoyle ou hydroxyalcanoyle de 2—24 carbones ou une chaîne alcénoyle de 3—24 carbones et le substituant Z est un groupe de solubilisation dans l'eau; en combinaison avec un support pharmaceutique, pour la préparation d'un agent immunostimulant.

5. Utilisation selon la revendication 4, caractérisée par le fait que le composé est le lipide X de formule:

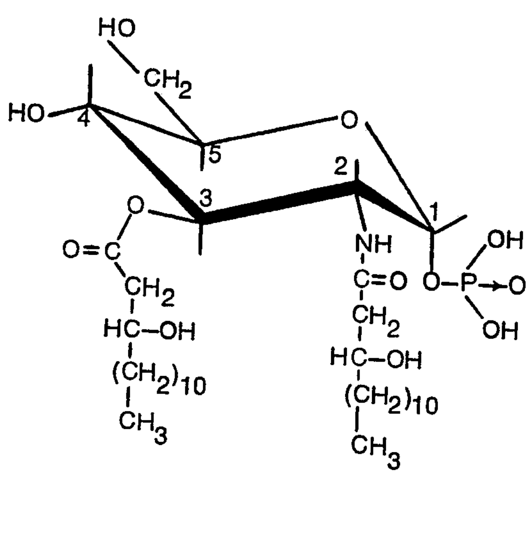

I

6. Utilisation selon la revendication 4, caractérisée par le fait que le composé est le lipide Y de formule:

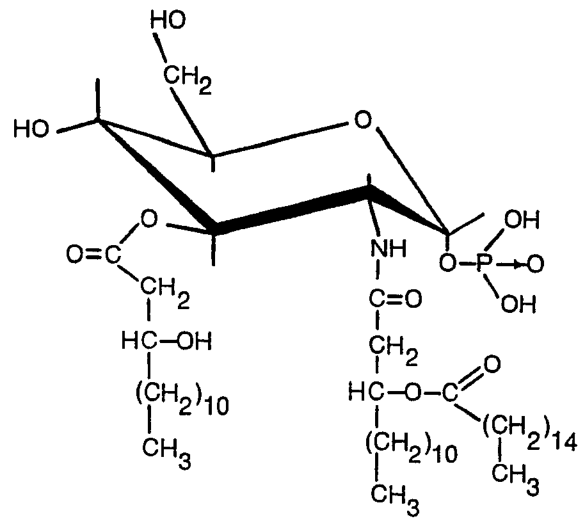

III

7. Procédé de stimulation de l'activité des cellules immunes d'un animal *in vitro*, qui comprend l'introduction, dans les cellules immunes de l'animal, d'un lipide X de formule:

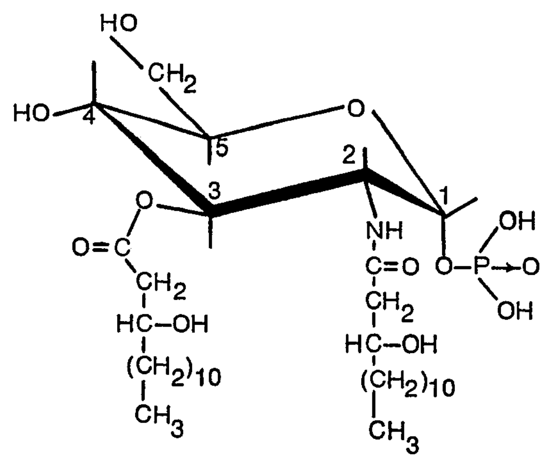

I

8. Procédé de stimulation de l'activité des cellules immunes d'un animal *in vitro*, qui comprend l'introduction, dans les cellules immunes de l'animal, d'un lipide Y de formule:

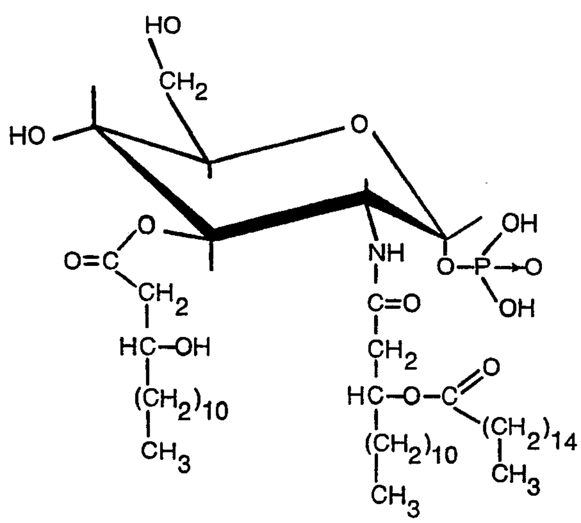

III

9. Composé de formule:

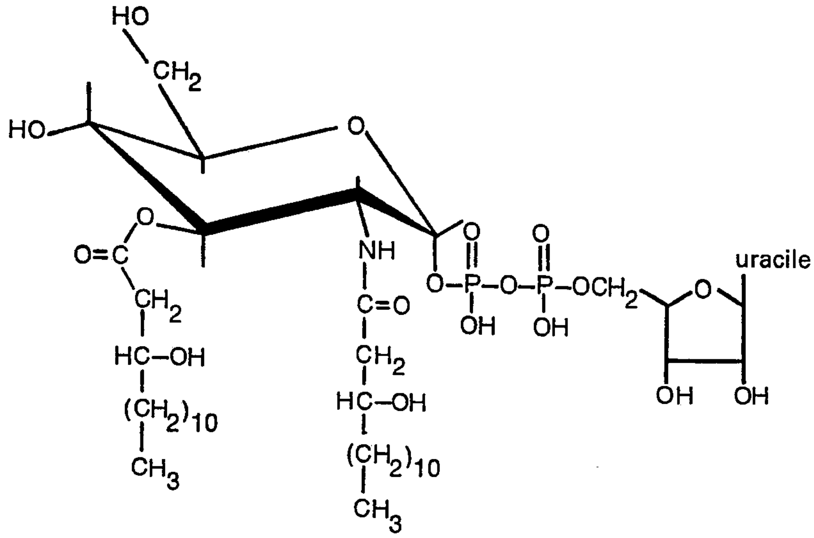

V

10. Composé de formule:

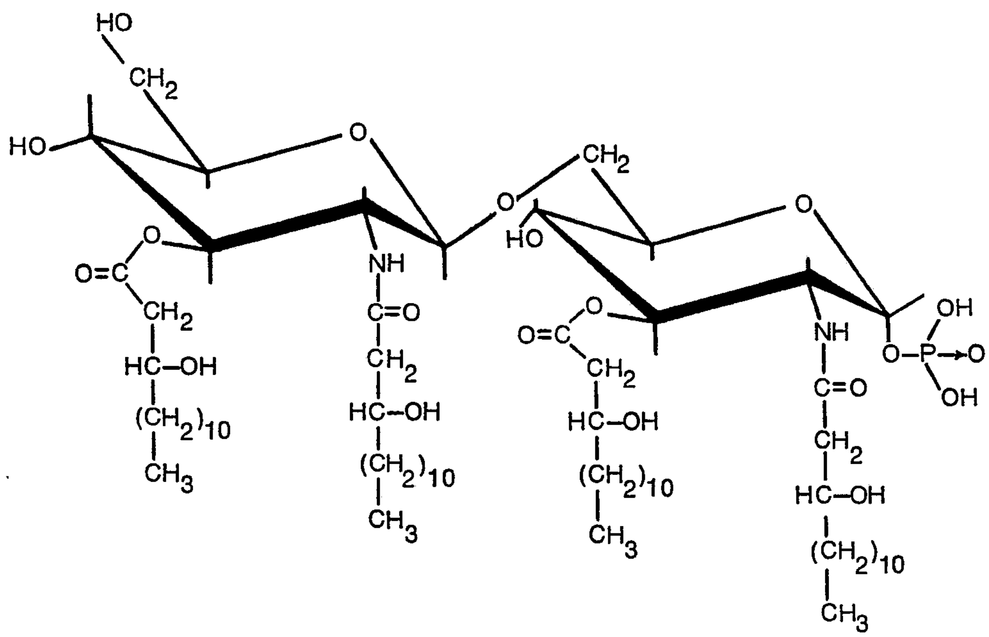

VI

qui peut étre issu de (V) et (I), en présence de préparations enzymatiques d'*E. coli*.

11. Composé de formule:

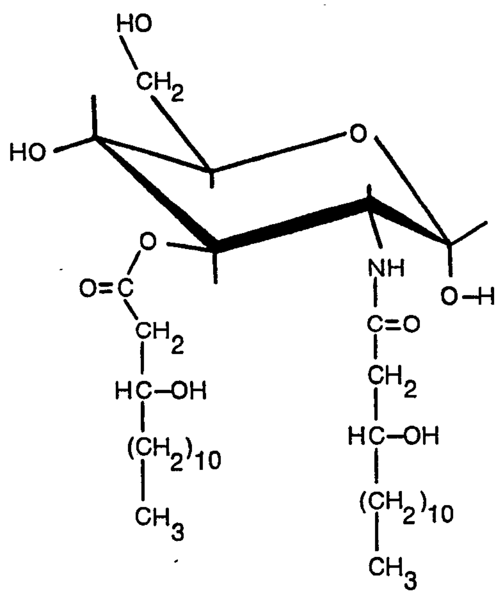

VII

12. Composé de formule:

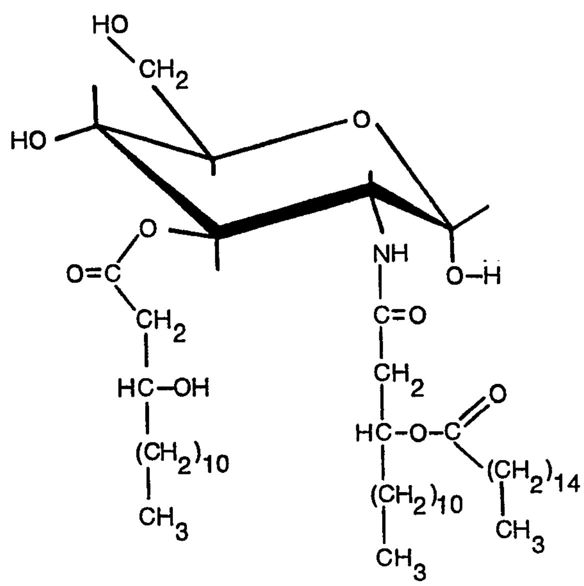

VIII